Europäisches Patentamt

European Patent Office

Office européen des brevets

(11) Veröffentlichungsnummer: **0 177 827 B1**

(12) **EUROPÄISCHE PATENTSCHRIFT**

(45) Veröffentlichungstag der Patentschrift: **18.11.93**

(51) Int. Cl.⁵: **C12N 15/62**, C07H 21/04, C12N 1/20, //(C12N1/20, C12R1:19)

(21) Anmeldenummer: **85112043.6**

(22) Anmeldetag: **23.09.85**

(54) **Synthetische Signalsequenz zum Transport von Proteinen in Expressionssystemen.**

(30) Priorität: **06.10.84 DE 3436818**

(43) Veröffentlichungstag der Anmeldung:
**16.04.86 Patentblatt 86/16**

(45) Bekanntmachung des Hinweises auf die Patenterteilung:
**18.11.93 Patentblatt 93/46**

(84) Benannte Vertragsstaaten:
**AT BE CH DE FR GB IT LI LU NL SE**

(56) Entgegenhaltungen:
**EP-A- 0 108 872**
**EP-A- 0 133 321**
**EP-A- 0 170 266**
**WO-A-84/03519**
**WO-A-84/04541**

**THE JOURNAL OF BIOCHEMISTRY, Band 97, Nr. 5, Mai 1985; T. MIYAKE et al., Seiten 1429-1436&NUM;**

(73) Patentinhaber: **HOECHST AKTIENGESELL-SCHAFT**

**D-65926 Frankfurt(DE)**

(72) Erfinder: **Engels, Joachim, Prof. Dr.**
**Feldbergstrasse 1**
**D-6242 Kronberg/Taunus(DE)**
Erfinder: **Leineweber, Michael, Dr.**
**Heimchenweg 74**
**D-6230 Frankfurt am Main 80(DE)**
Erfinder: **Uhlmann, Eugen, Dr.**
**Zum Talblick 31**
**D-6246 Glashütten/Taunus(DE)**
Erfinder: **Wetekam, Waldemar, Dr.**
**Zeilring 40/I**
**D-6239 Eppstein/Taunus(DE)**

EP 0 177 827 B1

**Beschreibung**

Proteine werden in der Zelle an den Ribosomen synthetisiert, die im Cytoplasma lokalisiert sind. Proteine, die aus dem Cytoplasma heraus transportiert werden, tragen am Aminoterminus eine relativ kurze Peptidkette, die beim Passieren durch die Cytoplasmanembran enzymatisch abgetrennt wird, wobei das "reife" Protein entsteht. Diese kurze Peptidsequenz wird als "Signalpeptid" oder auch als Prae- bzw. leader-Sequenz bezeichnet.

Es wurde schon für eine Vielzahl sekretorischer Proteine die am Aminoterminus angeordnete Signalsequenz charakterisiert. Sie besteht generell aus einer hydrophoben Region von etwa 10 bis 20 Aminosäuren, die als "core" bezeichnet wird, an deren Aminoterminus eine kurze Peptidsequenz ("pre-core") gebunden ist, die meist eine positiv geladene Aminosäure (oder mehrere) aufweist. Zwischen dem Carboxyterminus der hydrophoben Region und dem Aminoterminus des "reifen" transportierten Proteins liegt eine kurze Peptidsequenz ("post-core"), welche die Spaltstelle ("splice-site") enthält und für eine günstige räumliche Anordnung sorgt.

Aus der US-Patentschrift 4 411 994 ist bekannt, das Gen für ein zu exprimierendes Protein an ein bakterielles Gen zu koppeln, das für ein extracelluläres oder periplasmatisches Trägerprotein codiert, um so den Transport des gewünschten Proteins aus dem Cytoplasma zu bewerkstelligen. Für dieses Verfahren muß man ein wirtseigenes, bakterielles Gen für ein periplasmatisches, "outer membrane"- oder ein extracelluläres Protein isolieren. Dieses Gen wird dann mit einem Restriktionsenzym geschnitten, das Gen für das zu transportierende Protein in die gebildete Schnittstelle inseriert und die Wirtszelle mit einem Vektor transformiert, der das so gebildete Fusionsgen enthält. Die Isolierung des natürlichen Gens und seine Charakterisierung zur Auswahl geeigneter Schnittstellen ist außerordentlich aufwendig.

Erfindungsgemäß wird dieser Aufwand dadurch vermieden, daß von einer synthetischen Signalsequenz Gebrauch gemacht wird.

Die Erfindung betrifft deshalb eine synthetische Signalsequenz zum Transport von Proteinen in Expressionssystemen, die dadurch gekennzeichnet ist, daß die DNA im wesentlichen einer natürlichen Signalsequenz entspricht, aber eine oder mehrere Schnittstellen für Endonucleasen aufweist, die in der natürlichen DNA nicht enthalten sind. Weitere Aspekte der Erfindung und bevorzugte Ausgestaltungen sind im folgenden wiedergegeben bzw. in den Patentansprüchen niedergelegt.

Die DNA soll "im wesentlichen" der einer natürlichen Signalsequenz entsprechen. Hierunter ist zu verstehen, daß das exprimierte Signalpeptid mit dem natürlichen weitgehend oder völlig übereinstimmt, im letzteren Falle also lediglich auf der DNA-Ebene insofern ein Unterschied besteht, als die synthetische DNA mindestens eine Schnittstelle aufweist, die in der natürlichen DNA-Sequenz nicht enthalten ist. Dieser Einbau der erfindungsgemäßen Schnittstelle bedingt also eine mehr oder weniger große Abweichung von der natürlichen Sequenz, wobei unter Umständen auf Codons zurückgegriffen werden muß, die bekanntermaßen vom jeweiligen Wirtsorganismus weniger bevorzugt werden. Überraschenderweise ist hiermit jedoch kein Nachteil in der Expression verknüpft. Das gezielte "Maßschneidern" des synthetischen Gens bringt vielmehr so viele Vorteile mit sich, daß ein eventueller Nachteil durch den "unnatürlichen" Codon-Gebrauch im allgemeinen weit überkompensiert wird. Es hat sich sogar gezeigt, daß der Ersatz des Startcodons GTG, das im Gen der Alkalischen Phosphatase von E. coli vorkommt, durch ATG zu einer stark erhöhten Expression führt. Ein besonderer Vorteil der Erfindung liegt darin, daß die Wirtszelle weniger Ballast-Protein produzieren muß, da das zu exprimierende Gen direkt an das 3'-Ende der synthetischen DNA-Signalsequenz angeknüpft werden kann. Darüber hinaus ergeben sich insofern Vorteile, als sich bei der Konstruktion der synthetischen DNA an den Enden überstehende DNA-Sequenzen für bestimmte Restriktionserkennungsstellen vorsehen lassen, die eine Klonierung dieser Sequenz erlauben und im Falle unterschiedlicher Erkennungsstellen einen definierten Einbau in einen Klonierungsvektor gestatten. Hierdurch wird nach dem "Baukastenprinzip" ein Einbau in beliebige Vektoren ermöglicht.

Interne Erkennungsstellen für Restriktionsenzyme gestatten die Ankoppelung beliebiger homologer oder heterologer Gene im richtigen Leserahmen. Über diese internen Schnittstellen können auch auf einfache Weise Variationen in die DNA der Signalsequenzen eingeführt werden, die zu in der Natur nicht vorkommenden Praesequenzen führen.

Zweckmäßig werden diese internen Schnittstellen in die Bereiche vor und nach der hydrophoben Region gelegt, insbesondere in die "post-core"-Region, wobei die Spaltstelle und/oder deren Umgebung modifiziert werden kann. Es ist natürlich auch möglich, die "core"-Region in an sich bekannter Weise zu modifizieren.

Unter Berücksichtigung bekannter Regeln (G. von Heijne, J. Mol. Biol. 173 (1984) 243 - 251) kann über geeignete Schnittstellen im Genabschnitt, der für den carboxyterminalen Teil des Praepeptids codiert, die Signalpeptidase-Spaltstelle derart geplant werden, daß kein Fusionsprotein, sondern direkt das gewünschte, i.a. eukaryotische Peptid in nativer Form exprimiert wird. Gene natürlichen Ursprungs lassen eine solche Prozessierung im allgemeinen nicht zu.

Als Signalsequenzen kommen im Prinzip alle literaturbekannten Signalsequenzen (M.E.E. Watson; Nucleic Acids Res. 12 (1984), 5145 - 5164), Variationen derselben sowie daraus abgeleitete "idealisierte" Signalsequenzen (D. Perlman und H.O. Halvorson; J. Mol. Biol. 167 (1983), 391 - 409) in Betracht.

Bevorzugte Wirtsorganismen sind E. coli, Streptomyces, Staphylococcus-Arten wie S. aureus, Bacillus-Arten wie B. subtilis, B. amyloliquifaciens, B. cereus oder B. licheniformis, Pseudomonas, Saccharomyces, Spodoptera frugiperda und Zellinien höherer Organismen wie pflanzliche oder tierische Zellen.

Prinzipiell lassen sich alle diejenigen Proteine prokaryotischen oder eukaryotischen Ursprungs durch Transportexpression gewinnen, die membrangängig sind. Bevorzugt sind aber pharmazeutisch bedeutende Peptidprodukte wie Hormone, Lymphokine, Interferone, Blutgerinnungsfaktoren und Vakzine, die in der Natur auch als Peptide mit einer aminoterminalen Praesequenz codiert sind. Diese eukaryotische Praesequenz wird jedoch in der Regel in den prokaryotischen Wirtsorganismen nicht von den wirtseigenen Signalpeptidasen abgespalten.

In E. coli sind die Gene für die periplasmatischen und "outer membrane"-Proteine zur Transportexpression geeignet, wobei die erstgenannten das Produkt ins Periplasma, die letztgenannten aber eher an die äußere Membran dirigieren.

Als Beispiel wird die DNA-Signalsequenz des periplasmatischen Proteins Alkalische Phosphatase, das in E. coli hochexprimierbar ist, angeführt, ohne daß die Erfindung darauf beschränkt wäre.

Im folgenden ist die Prae-Sequenz einschließlich der ersten zwanzig Aminosäuren der Alkalischen Phosphatase von E. coli dargestellt:

```
     1                5                    10
  Met-Lys-Gln-Ser-Thr-Ile-Ala-Leu-Ala-Leu-Leu-Pro-Leu-Leu-

    15                  20                   25
  Phe-Thr-Pro-Val-Thr-Lys-Ala-Arg-Thr-Pro-Glu-Met-Pro-Val-
                          ↑
      30                  35                   40
  Leu-Glu-Asn-Arg-Ala-Ala-Gln-Gly-Asn-Ile-Thr-Ala-Pro


   ↑= bevorzugte Spaltstelle der Signalpeptidase
```

Es hat sich gezeigt, daß bis zu etwa 40, meist etwa 20 zusätzliche Aminosäuren des reifen Proteins für eine korrekte Prozessierung ausreichend sind. In vielen Fällen genügen jedoch auch weniger zusätzliche Aminosäuren, beispielsweise etwa 10, vorteilhaft etwa 5. Da eine kürzere Proteinkette eine geringere Beanspruchung des Protein-Biosyntheseapparats der Wirtszelle bedingt, ist eine vorteilhafte Ausführungsform der Erfindung in der DNA-Sequenz I (Anhang) niedergelegt, die für die Praesequenz der Alkalischen Phosphatase sowie zusätzlicher 5 Aminosäuren des reifen Proteins codiert. Die DNA-Sequenz I entspricht bis auf einige Triplett-Variationen - nämlich solchen, die singuläre Restriktionsenzym-Schnittstellen einführen und das Startcodon GTG durch ATG ersetzen - der natürlichen Sequenz der Alkalischen Phosphatase. An den Enden des codierenden Stranges befinden sich "überhängende" DNA-Sequenzen entsprechend der Restriktionsendonuclease EcoR I, die den Einbau in gängige Klonierungsvektoren, beispielsweise die handelsüblichen Plasmide wie pBR 322, pUC 8 oder pUC 12 erlauben. Zusätzlich wurden innerhalb des Gens der DNA-Sequenz I eine Reihe von weiteren singulären Schnittstellen für Restriktionsenzyme eingebaut, die einerseits die Ankoppelung heterologer Gene an der richtigen Stelle und im gewünschten Leserahmen ermöglichen und andererseits auch die Durchführung von Variationen erlauben:

| Restriktionsenzym | Schnitt nach Nucleotid-Nr. (im codierenden Strang) |
| --- | --- |
| Sau 3 A | 19 |
| Pvu I | 22 |
| Hpa II | 54 ) (im natürlichen |
| Nci I | 54 ) Gen vorhanden) |
| Alu I | 66 |
| Hph I | 68 |
| Ava II | 70 |

Es ist selbstverständlich auch möglich, die überhängenden Sequenzen so zu konstruieren, daß sie verschiedenen Restriktionsenzymen entsprechen, was dann den Einbau in geeignete Vektoren in definierter Orientierung erlaubt. Der Fachmann wird hierbei abwägen, ob der mit dem Konstruieren des Gens und seinem gezielten Einbau verbundene Aufwand schwerer wiegt als die zusätzliche Selektionsarbeit, die mit einem Einbau in beiden Orientierungsrichtungen bei identischen überstehenden Enden verbunden ist.

Die DNA-Sequenz I läßt sich aus 6 Oligonucleotiden mit einer Länge von 26 - 31 Basen aufbauen, indem diese zunächst chemisch synthetisiert und dann über "sticky ends" von 6 Nucleotiden enzymatisch verknüpft werden. Der Einbau des synthetischen Gens in Klonierungsvektoren, beispielsweise in die genannten handelsüblichen Plasmide, erfolgt in an sich bekannter Weise.

Als Beispiel für die Expression eines eukaryotischen Gens in E. coli mit Hilfe einer erfindungsgemäßen Praesequenz wird im folgenden die Synthese von Affen-Proinsulin beschrieben: Man konstruiert eine DNA-Sequenz, bei der hinter einer chemisch-synthetischen Regulationsregion, bestehend aus einem bakteriellen Promotor, einem lac-Operator und einer ribosomalen Bindungsstelle (Deutsche Patentanmeldung P 34 30 683.8), 6 - 14 Nucleotide von der Ribosomen-Bindungsstelle entfernt, an eine anschließende Erkennungssequenz für EcoR I die DNA-Sequenz I und daran anschließend das Proinsulingen (W. Wetekam et al., Gene 19 (1982)179-183) angeordnet ist. Das exprimierte Proinsulin-Fusionspeptid enthält an Aminoterminus noch 9 zusätzliche Aminosäuren, die enzymatisch oder chemisch abgespalten werden können.

Der Einbau des synthetischen Gens in pUC 8 sowie die Konstruktion von Expressionsplasmiden, die eukaryotische Gene an die DNA-Sequenz I gekoppelt enthalten, erfolgt in an sich bekanner Weise. Hierzu kann auf das Lehrbuch von Maniatis (Molecular Cloning, Maniatis et al., Cold Spring Harbor, 1982) verwiesen werden. Die Transformation der so erhaltenen Hybridplasmide in geeignete Wirtsorganismen, vorteilhaft E. coli, ist ebenfalls an sich bekannt und in dem vorstehend genannten Lehrbuch eingehend beschrieben. Die Gewinnung der exprimierten Proteine und deren Reinigung ist ebenfalls beschrieben.

In den folgenden Beispielen werden noch einige Ausgestaltungen der Erfindung im einzelnen erläutert, woraus sich die Vielzahl der möglichen Abwandlungen (und Kombinationen) für den Fachmann ergeben. Prozentangaben beziehen sich hierbei auf das Gewicht, wenn nichts anderes angegeben ist.

Beispiele

1. Chemische Synthese eines einzelsträngigen Oligonucleotids

Am Beispiel des Genbausteins Ia, der die Nucleotide 1 - 29 des codierenden Strangs umfaßt, wird die Synthese der Genbausteine erläutert. Nach bekannten Methoden (M.J. Gait et al., Nucleic Acids Res. 8 (1980) 1081 - 1096)) wird das am 3'-Ende stehende Nucleosid, im vorliegenden Falle also Guanosin (Nucleotid Nr. 29), an Kieselgel (FRACTOSIL, Firma Merck) über die 3'-Hydroxyfunktion covalent gebunden. Hierzu wird zunächst das Kieselgel unter Abspaltung von Ethanol mit 3-(Triethoxysilyl)-propylamin umgesetzt, wobei eine Si-O-Si-Bindung entsteht. Das Guanosin wird als $N^{2'}$-Isobutyryl-3'-O-succinoyl-5'-dimethoxytritylether in Gegenwart von Paranitrophenol und N,N'-Dicyclohexylcarbodiimid mit dem modifizierten Träger umgesetzt, wobei die freie Carboxygruppe der Succinoylgruppe den Aminorest der Propylamingruppe acyliert.

In den folgenden Syntheseschritten wird die Basenkomponente als 5'-O-Dimethoxytrityl-nucleosid-3'-phosphorigsäure-monomethylester-dialkylamid oder -chlorid eingesetzt, wobei das Adenin als $N^6$-Benzoyl-

Verbindung, das Cytosin als $N^4$-Benzoyl-Verbindung, das Guanin als $N^2$-Isobutyryl-Verbindung und das keine Aminogruppe enthaltende Thymin ohne Schutzgruppe vorliegen.

50 mg des polymeren Trägers, der 2 $\mu$mol Guanosin gebunden enthält, werden nacheinander mit den folgenden Agentien behandelt:

a) Nitromethan

b) gesättigte Zinkbromidlösung in Nitromethan mit 1 % Wasser

c) Methanol

d) Tetrahydrofuran

e) Acetonitril

f) 40 $\mu$mol des entsprechenden Nucleosidphosphits und 200 $\mu$mol Tetrazol in 0,5 ml wasserfreiem Acetonitril (5 Minuten)

g) 20 % Acetanhydrid in Tetrahydrofuran mit 40 % Lutidin und 10 % Dimethylaminopyridin (2 Minuten)

h) Tetrahydrofuran

i) Tetrahydrofuran mit 20 % Wasser und 40 % Lutidin

j) 3 % Jod in Kollidin/Wasser/Tetrahydrofuran im Volumenverhältnis 5 : 4 : 1

k) Tetrahydrofuran und

l) Methanol.

Unter "Phosphit" wird hierbei der Desoxyribose-3'-monophosphorigsäure-monomethylester verstanden, wobei die dritte Valenz durch Chlor oder eine tertiäre Aminogruppe, beispielsweise einen Morpholinorest, abgesättigt ist. Die Ausbeuten der einzelnen Syntheseschritte können jeweils nach der Detritylierungsreaktion (b) spektrophotometrisch durch Messung der Absorption des Dimethoxytritylkations bei der Wellenlänge von 496 nm bestimmt werden.

Nach abgeschlossener Synthese des Oligonucleotids werden die Methylphosphatschutzgruppen des Oligomers mit Hilfe von p-Thiokresol und Triethylamin abgespalten. Anschließend wird durch 3-stündige Behandlung mit Ammoniak das Oligonucleotid vom festen Träger abgetrennt. Eine 2- bis 3-tägige Behandlung der Oligomeren mit konzentriertem Ammoniak spaltet die Aminoschutzgruppen der Basen quantitativ ab. Das so erhaltene Rohprodukt wird durch Hochdruckflüssigkeitschromatographie (HPLC) oder durch Polyacrylamid-Gelelektrophorese gereinigt.

Ganz entsprechend werden auch die übrigen Genbausteine Ib - If synthetisiert, deren Nucleotidfolge aus der DNA-Sequenz II (Anhang) hervorgeht.

2. Enzymatische Verknüpfung der einzelsträngigen Oligonucleotide zur DNA-Sequenz I

Die endständigen Oligonucleotide Ia und If werden nicht phosphoryliert. Damit wird eine Oligomerisation über die überhängenden Enden vermieden. Zur Phosphorylierung der Oligonucleotide Ib, Ic, Id und Ie werden jeweils 1 nmol dieser Verbindungen mit 5 nmol Adenosintriphosphat und vier Einheiten T4-Polynucleotid-Kinase in 20 $\mu$l 50 mM Tris-HCl-Puffer (pH 7,6), 10 mM Magnesiumchlorid und 10 mM Dithiothreitol (DTT) 30 Minuten bei 37 °C behandelt. Das Enzym wird durch fünfminütiges Erhitzen auf 95 °C desaktiviert. Anschließend werden die Oligonucleotide Ia bis If vereinigt und zum Doppelstrang hybridisiert, indem man sie in einer 20 mM KCl-Lösung erhitzt und dann langsam (im Laufe von 2 Stunden) auf 16 °C abkühlt. Die Ligation zum DNA-Fragment gemäß DNA-Sequenz I erfolgt durch Reaktion in 40 $\mu$l 50 mM Tris-HCl-Puffer (20 mM Magnesiumchlorid und 10 mM DTT) mit Hilfe von 100 Einheiten T4-DNA-Ligase bei 15 °C im Laufe von 18 Stunden.

Die Reinigung des Genfragments erfolgt durch Gelelektrophorese auf einem 10 %igen Polyacrylamidgel (ohne Harnstoffzusatz, 20 x 40 cm, 1 mm Dicke), wobei als Markersubstanz $\phi$X 174 DNA (Fa. BRL), geschnitten mit Hinf I, oder pBR 322, geschnitten mit Hae III, dient.

3. Einbau des Genfragmentes in pUC 8

Das handelsübliche Plasmid pUC 8 wird in bekannter Weise mit der Restriktionsendonuclease EcoR I nach den Angaben des Herstellers geöffnet. Der Verdauungsansatz wird auf einem 5 %igen Polyacrylamidgel durch Elektrophorese in bekannter Weise aufgetrennt und die DNA durch Anfärben mit Ethidiumbromid oder durch radioaktive Markierung ("Nick-Translation" nach Maniatis, a.a.O.) sichtbar gemacht. Die Plasmidbande wird anschließend aus dem Acrylamidgel ausgeschnitten und elektrophoretisch vom Polyacrylamid abgetrennt.

4. Einbau der DNA-Sequenz I in ein Expressionsplasmid

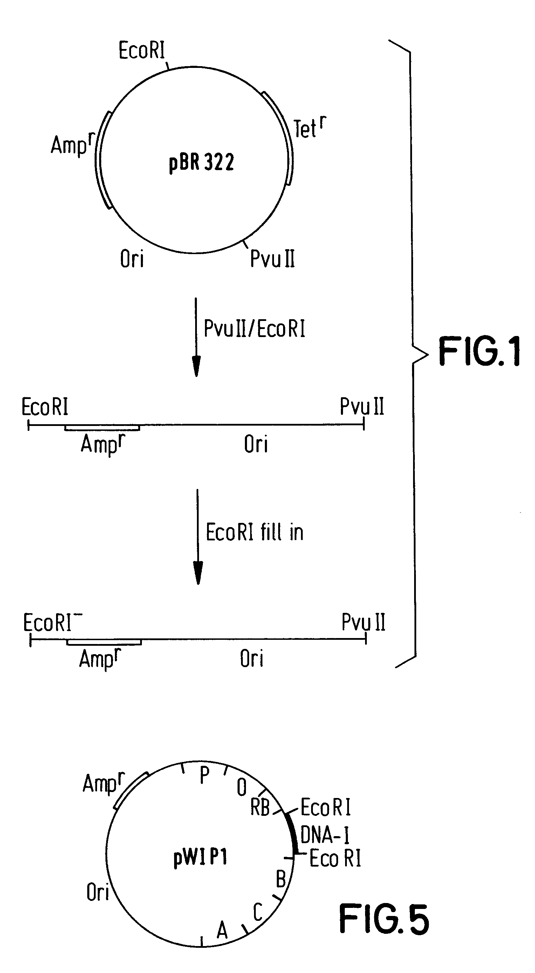

Das Expressionsplasmid pWI 6 mit der Information für das Affen-Proinsulin wird wie folgt konstruiert: 10 μg des Plasmids pBR 322 werden mit den Restriktionsendonucleasen Eco RI/Pvu II verdaut und anschließend durch eine "fill-in"-Reaktion mit Klenow-Polymerase an der Eco RI-Schnittstelle aufgefüllt. Nach gelelektrophoretischer Auftrennung in einem 5 %igen Polyacrylamidgel läßt sich das Plasmidfragment von 2293 Bp Länge durch Elektroelution gewinnen (Figur 1).

Aus dem Plasmid pBR 322 mit integrierter Affenpräproinsulin-DNA (Wetekam et al., Gene 19 (1982) 179 - 183) wird diese durch Verdauung mit den Restriktionsendonucleasen Hind III und Mst I (als Fragment von etwa 1250 Bp) isoliert und in das Plasmid pUC 9 wie folgt rekloniert: Das Plasmid pUC 9 wird mit dem Enzym Bam HI gespalten, die Schnittstelle in einer Standard-"fill-in"-Reaktion mit Klenow-Polymerase ("large fragment") aufgefüllt, mit dem Restriktionsenzym Hind III nachgeschnitten und die DNA gelelektrophoretisch in einem 5%igen Polyacrylamidgel von den übrigen DNA-Fragmenten abgetrennt. In das geöffnete Plasmid wird das isolierte Insulin-DNA-Fragment von etwa 1250 Bp Länge integriert.

Zur Abtrennung der "untranslated region" und der Praesequenz wird das so modifizierte pUC 9-Plasmid mit Hae III verdaut und das 143 Bp lange Fragment zur Abspaltung der letztenbeiden Nucleotide aus der Praesequenz unter limitierenden Enzymbedingungen mit Bal 31 verdaut. Man erhält so am Amino-Terminus als erstes Codon TTT, das für Phenylalanin als erste Aminosäure der B-Kette steht.

An dieses Fragment wird nun ein für Eco RI spezifischer Adaptor in einer "blunt-end" Ligationsreaktion ligiert:

```
a) 5'      AAT TAT GAA TTC GCA ATG
   Eco RI      TA CTT AAG CGT TAC


b) 5'      AAT TAT GAA TTC GCA AGA
   Eco RI      TA CTT AAG CGT TCT
```

Um eine Polymerisierung der Adaptoren zu vermeiden, wurden diese unphosphoryliert in die Ligationsreaktion eingesetzt (was in den Figuren mit Eco RI⁻ angedeutet ist, ebenso wie, z. B. durch Auffüllen, inaktivierte Erkennungssequenzen). Der Adaptor a) weist am Ende ein Codon für Methionin auf, der Adaptor b) das Codon für Arginin.

Nach der Variante a) wird somit ein Genprodukt erhalten, bei dem durch Bromcyanspaltung eine Abtrennung des bakteriellen Anteils möglich ist, während die Variante b) eine Trypsinspaltung erlaubt.

Das Ligationsprodukt wird mit Mbo II verdaut. Nach gelelektrophoretischer Auftrennung erhält man ein DNA-Fragment von 79 Bp Länge mit der Information für die Aminosäuren Nr. 1 bis 21 der B-Kette.

Das Gen für die restliche Information des Proinsulinmoleküls (einschließlich einer G-C-Sequenz aus der Klonierung und 21 Bp aus dem pBR 322 im Anschluß an das Stop-Codon) erhält man aus dem pUC 9-Plasmid mit der kompletten Präproaffeninsulin-Information durch Verdauung mit Mbo II/Sma I und Isolierung eines DNA-Fragments von etwa 240 Bp Länge. Durch Ligieren der beiden Proinsulin-Fragmente erhält man das korrekte Ligationsprodukt von etwa 320 Bp Länge (inklusive des Adaptors von 18 Bp). Dieses so konstruierte Proinsulin-DNA-Fragment kann nun mit einer Regulationsregion über die Eco RI-negative Schnittstelle zusammenligiert werden.

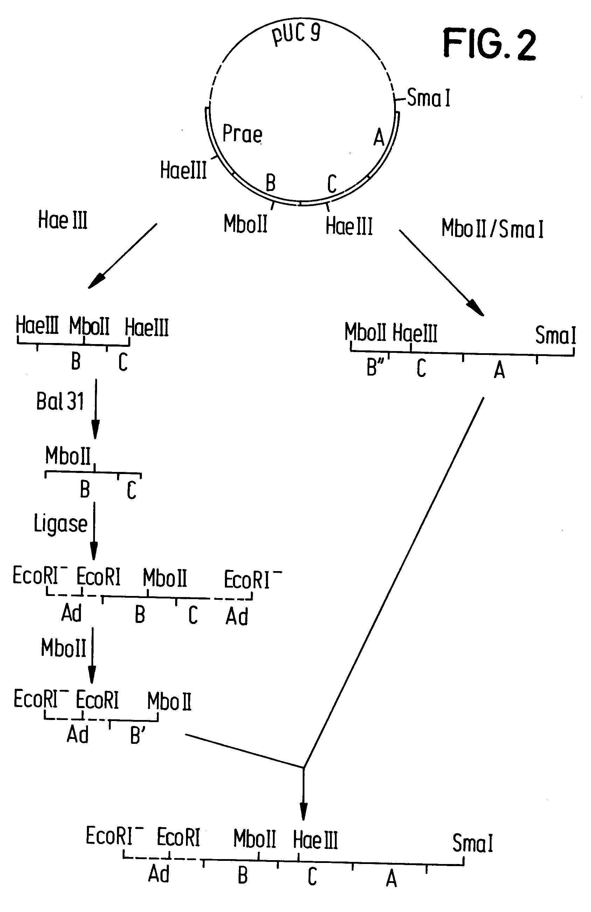

Die gesamte Reaktionsfolge zeigt die Fig. 2, in der A, B und C die DNA für die Jeweiligen Peptidketten des Proinsulinmoleküls, Ad den (dephosphorylierten) Adaptor (a oder b) und Prae die DNA für die Praesequenz des Affenpraeproinsulins bedeuten.

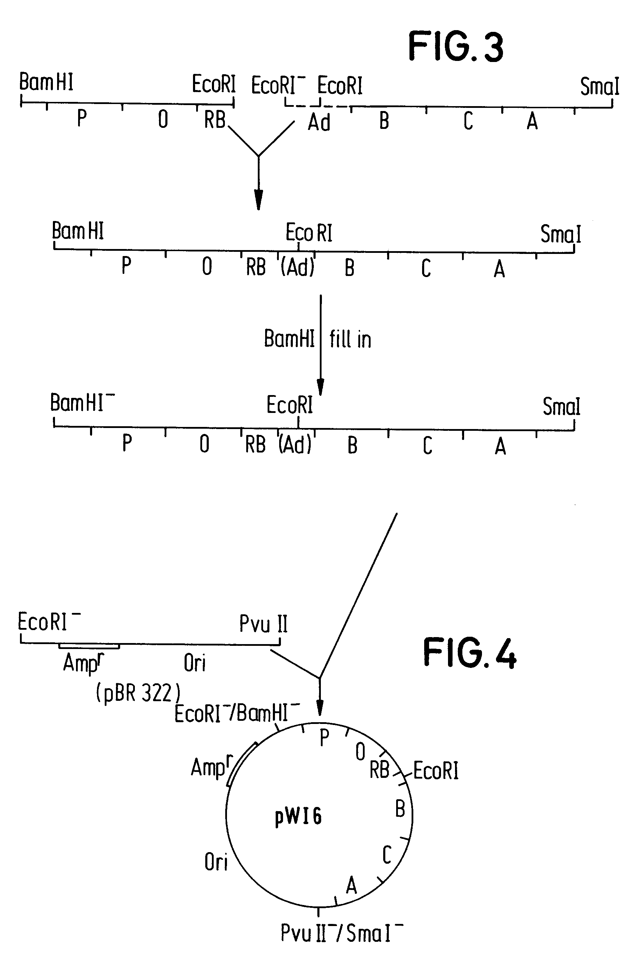

Eine chemisch-synthetische Regulationsregion bestehend aus einer Erkennungssequenz für Bam HI, dem lac-Operator (O), einem bakteriellen Promotor (P) und einer ribosomalen Bindungsstelle (RB) mit einem ATG-Start-Codon, 6 bis 14 Nucleotide von der RB entfernt, und einer anschließenden Erkennungssequenz für Eco RI (Figur 3) wird über die gemeinsame Eco RI-Überlappungsregion mit dem nach dem vorstehenden Beispiel erhaltenen Proinsulingen-Fragment ligiert. Vorteilhaft wählt man die folgende synthetische Regulationsregion (DNA-Sequenz IIa aus der Tabelle 2, entsprechend der deutschen Patentanmeldung P 34 30 683.8):

```
5'  GATCCTAAATAAATTCTTGACATTTTTTAAA  3'
3'     GATTTATTTAAGAACTGTAAAAAATTT  5'


   (Bam HI)                          P


5'  TAATTTGGTATAATGTGTGGAATTGTGAGCG  3'
3'  ATTAAACCATATTACACACCTTAACACTCGC  5'


                        O


5'  GAATAACAATTTCACAGAGGATCTAG       3'
3'  CTTATTGTTAAAGTGTCTCCTAGATCTTAA   5'


                   RB       (Eco RI)
```

Ebenso können die anderen in der Tabelle 2 genannten synthetischen Regulationsregionen eingesetzt werden. Es ist aber auch möglich, eine literaturbekannte natürliche oder abgeleitete (Perlman et al., a.a.O.) Signalsequenz zu wählen.

TABELLE 2

Synthetische Regulationsregion (codierender Strang):

5' GGATCCTAAATAAAATTCTTGACATTT1TTAA2TAATTTGGTATAATGT3T

4GAATTG5GAGCG6T7ACAATT8C9C10G11G12T13TA14TT15 (ATG) 3'

| | | | |
|---|---|---|---|
| 1 = T oder G | | 11 = AG oder GA | |
| 2 = A oder C | | 12 = A oder G | |
| 3 = G oder C | | 13 = C oder T | |
| 4 = G oder A | | 14 = GAA oder AGC | |
| 5 = T oder C | | 15 = C oder direkte Bindung | |
| 6 = C, GA oder GAA | | | |
| 7 = A oder C | | | |
| 8 = T oder direkte Bindung | | | |
| 9 = A oder TAGA | | | |
| 10 = A, TTTAAA, AAGCTT oder AAGCTA | | | |

DNA-Sequenzen IIa-h:

| | 1 | 2 | 5 | 6 | 7 | 8 | 10 | 11 | 12 | 13 | 14 | 15 |
|---|---|---|---|---|---|---|---|---|---|---|---|---|
| IIa | T | A | T | GAA | A | T | A | AG | A | C | GAA | C |
| b | T | A | T | GAA | A | T | TTTAAA | AG | A | C | GAA | C |
| c | G | C | T | GAA | A | T | TTTAAA | AG | A | C | GAA | C |
| d | G | C | T | GAA | A | - | AAGCTT | AG | A | C | GAA | C |
| e | G | C | C | GAA | C | - | AAGCTT | AG | A | C | GAA | C |
| f | G | C | T | C | C | - | AAGCTT | AG | A | C | GAA | C |
| g | G | C | T | GA | C | - | AAGCTA | GA | G | T | AGC | - |
| h | G | C | T | GA | C | - | AAGCTA | | | | | |

(3 = G, 4 = G, 9 = A)

Nach einer Doppelverdauung mit Sma I/Bam HI und einer "fill-in" Reaktion der Bam HI Schnittstelle mit dem Klenow-Fragment wird das Ligierungsprodukt (ungefähr 420 Bp) gelelektrophoretisch isoliert.

Das so erhaltene Fragment läßt sich anschließend über eine "blunt-end" Ligierung in das pBR 322-Teilplasmid gemäß Figur 1 hineinligieren (Figur 4). Man erhält das Hybridplasmid pWI 6.

Nach Transformation in den E. coli-Stamm HB 101 und Selektion auf Ampicillin-Platten wurde die Plasmid-DNA individueller Klone auf die Integration eines 420 Bp-Fragments mit der Regulationsregion und dem Bal 31-verkürzten Proinsulingens getestet. Zum Nachweis der korrekten Verkürzung des Proinsulingens durch Bal 31 (Figur 2) wurden die Plasmide mit dem integrierten Proinsulingenfragment von der Eco RI-Schnittstelle aus sequenziert. Von 60 sequenzierten Klonen hatten drei die gewünschte Verkürzung von zwei Nucleotiden (Figur 4).

1 µg des Plasmides pWI 6 wird mit dem Restriktionsenzym Eco RI geschnitten und anschließend in Gegenwart von 30 ng DNA-Sequenz I bei 16°C in 6 Stunden zusammenligiert. Nach Transformation in E. coli HB 101 werden aus individuellen Klonen Plasmide isoliert und mit Hilfe einer Restriktionsenzymanalyse auf die Integration der DNA-Sequenz I getestet. 7 % aller Klone enthielten das Plasmid pWI 6 mit der DNA-Sequenz I integriert.

Die Richtung dieser Integrationsreaktion läßt sich mit Standardmethoden der Restriktionsenzymanalyse über eine Doppelverdauung mit Hind III/Pvu I eindeutig ermitteln. Das Plasmid pWI 6 mit einer DNA-Sequenz I in korrekter Leserichtung zum Proinsulingen integriert ist als pWIP 1 in Figur 5 dargestellt.

Dieses Plasmid kann anschließend in verschiedene E. coli-Stämme hineintransformiert werden, um die Synthesekapazität der einzelnen Stämme auszutesten.

Die Expression der Praesequenz-Proinsulingen-Fusion in E. coli wird folgendermaßen bestimmt:

1 ml einer mit IPTG (Isopropyl-$\beta$-D-thiogalactopyranosid) induzierten Bakterienkultur bei einer optischen Dichte $OD_{600}$ von 1,0 und einer Induktionsdauer von 1 Stunde werden mit PMSF (Phenylmethylsulfonylfluorid) in einer Endkonzentration von $5 \times 10^{-4}$ M gestoppt, in Eis gekühlt und abzentrifugiert. Der Zellniederschlag wird anschliessend in 1 ml Puffer (10 mM Tris-HCl, pH 7,6; 40 mM NaCl) gewaschen, abzentrifugiert und in 200 µl Puffer (20 % Saccharose; 20 mM Tris-HCl, pH 8,0; 2 mM EDTA) resuspendiert, 10 Minuten bei Zimmertemperatur inkubiert, abzentrifugiert und sofort in 500 µl $H_2O$ bidest. resuspendiert. Nach 10 Minuten Inkubationszeit in Eis werden die "Schock-lysierten" Bakterien abzentrifugiert und der Überstand eingefroren. Dieser Überstand wird in einem Standard-Insulin-RIA (Amersham) auf Proinsulingehalt getestet.

Der Bakterienniederschlag wird noch einmal in 200 µl Lysozympuffer (20 % Saccharose; 2 mg/ml Lysozym; 20 mM Tris-HCl, pH 8,0; 2 mM EDTA) resuspendiert, 30 Minuten in Eis inkubiert, 3 mal 10 Sekunden mit Ultraschall behandelt und anschließend abzentrifugiert. Der so resultierende Überstand wird als Plasmafraktion auf Gehalt an Proinsulin in einem Radioimmunoassay getestet.

Individuelle Bakterienklone, welche das Plasmid pWIP 1 enthalten, wurden auf ihre Synthesekapazität und ihre Transportfähigkeit des Proinsulin-Praesequenz-Produktes untersucht. Es konnte gezeigt werden, daß sämtliche Bakterienklone in erwarteter Weise das produzierte Proinsulin zu ungefähr 90 % in den periplasmatischen Raum transportierten. Ungefähr 10 % der Proinsulin-RIA-Aktivität waren noch in der Plasmafraktion zu finden.

DNA-Sequenz I

| Triplett Nr. | | | | | | | | 1 | 2 | 3 |
|---|---|---|---|---|---|---|---|---|---|---|
| Aminosäure Nr. | | | | | | | | Met | Lys | Gln |
| Nucleotid Nr. | | | | | | | 5 | | 10 | |
| Codierender Strang | | | 5' | AA | TTC | ATG | AAA | CAA | | |
| nichtcod. Strang | | | 3' | | G | TAC | TTT | GTT | | |

| 4 | 5 | 6 | 7 | 8 | 9 | 10 | 11 | 12 | 13 |
|---|---|---|---|---|---|---|---|---|---|
| Ser | Thr | Ile | Ala | Leu | Ala | Leu | Leu | Pro | Leu |

| 15 | 20 | | 25 | | 30 | 35 | | 40 | |
|---|---|---|---|---|---|---|---|---|---|
| AGC | ACG | ATC | GCA | CTG | GCA | CTC | TTA | CCG | TTA |
| TCG | TGC | TAG | CGT | GAC | CGT | GAG | AAT | GGC | AAT |

| 14 | 15 | 16 | 17 | 18 | 19 | 20 | 21 | 22 | 23 |
|---|---|---|---|---|---|---|---|---|---|
| Leu | Phe | Thr | Pro | Val | Thr | Lys | Ala | Arg | Thr |

| 45 | 50 | | 55 | | 60 | 65 | | 70 | |
|---|---|---|---|---|---|---|---|---|---|
| CTG | TTT | ACC | CCG | GTG | ACA | AAA | GCT | CGG | ACC |
| GAC | AAA | TGG | GGC | CAC | TGT | TTT | CGA | GCC | TGG |

| 24 | 25 | 26 |
|---|---|---|
| Pro | Glu | Met |

| 75 | 80 | | 84 | | |
|---|---|---|---|---|---|
| CCA | GAA | ATG | G | | 3' |
| GGT | CTT | TAC | CTT | AA | 5' |

DNA-Sequenz II:

```
                   ◄─────────────────────── Ia ──────────────────────────►
5'  AA  TTC    ATG    AAA    CAA    AGC    ACG    ATC    GCA    CTG
3'         G   TAC    TTT    GTT    TCG    TGC    TAG    CGT    GAC
Eco RI  ◄────────────────────────────────── Ib ───────────────────────
```

```
      ◄──────────────────────── Ic ────────────────────────►
GCA    CTC    TTA    CCG    TTA    CTG    TTT    ACC    CCG
CGT    GAG    AAT    GGC    AAT    GAC    AAA    TGG    GGC
───────────────────►  ◄─────────────────── Id ──────────────────────
```

```
      ◄──────────────────────── Ie ────────────────────────►  Eco RI
GTG    ACA    AAA    GCT    CGG    ACC    CCA    GAA    ATG    G
CAC    TGT    TTT    CGA    GCC    TGG    GGT    CTT    TAC    CTT  AA
───────────────────►  ◄─────────────────── If ──────────────────────►
```

**Patentansprüche**

**Patentansprüche für folgende Vertragsstaaten : BE, CH, DE, FR, GB, IT, LI, LU, NL, SE**

1.  Synthetische DNA zum Transport von Proteinen in Expressionssystemen, dadurch gekennzeichnet, daß die DNA für eine natürliche Signalsequenz codiert, aber eine oder mehrere Schnittstellen für Endonucleasen aufweist, die in der natürlichen DNA nicht enthalten sind, und daß diese DNA am 3'-Ende mindestens 5 bis zu etwa 40 der aminoterminalen codierenden Codons desjenigen Strukturgens enthält, das natürlicherweise auf die Signalsequenz folgt, woran sich die Aminosäurefolge des gewünschten Proteins anschließt.

2.  DNA nach Anspruch 1, dadurch gekennzeichnet, daß sie interne Schnittstellen vor und/oder nach der hydrophoben Region enthält.

3.  DNA nach Anspruch 1 oder 2, dadurch gekennzeichnet, daß sie im wesentlichen für die natürliche Signalsequenz der Alkalischen Phosphatase von E. coli codiert.

4.

## DNA-Sequenz I

| Triplett Nr. | | | | 1 | 2 | 3 |
|---|---|---|---|---|---|---|
| Aminosäure Nr. | | | | Met | Lys | Gln |
| Nucleotid Nr. | | | 5 | · 10 | | |
| Codierender Strang | 5' | AA | TTC | ATG | AAA | CAA |
| nichtcod. Strang | 3' | | G | TAC | TTT | GTT |

| 4 | 5 | 6 | 7 | · 8 | 9 | 10 | 11 | 12 | 13 |
|---|---|---|---|---|---|---|---|---|---|
| Ser | Thr | Ile | Ala | Leu | Ala | Leu | Leu | Pro | Leu |

| 15 | 20 | | 25 | | 30 | 35 | | 40 | |
|---|---|---|---|---|---|---|---|---|---|
| AGC | ACG | ATC | GCA | CTG | GCA | CTC | TTA | CCG | TTA |
| TCG | TGC | TAG | CGT | GAC | CGT | GAG | AAT | GGC | AAT |

| 14 | 15 | 16 | 17 | 18 | 19 | 20 | 21 | 22 | 23 |
|---|---|---|---|---|---|---|---|---|---|
| Leu | Phe | Thr | Pro | Val | Thr | Lys | Ala | Arg | Thr |

| 45 | 50 | | 55 | | 60 | 65 | | 70 | |
|---|---|---|---|---|---|---|---|---|---|
| CTG | TTT | ACC | CCG | GTG | ACA | AAA | GCT | CGG | ACC |
| GAC | AAA | TGG | GGC | CAC | TGT | TTT | CGA | GCC | TGG |

| 24 | 25 | 26 |
|---|---|---|
| Pro | Glu | Met |

| 75 | 80 | | 84 | | |
|---|---|---|---|---|---|
| CCA | GAA | ATG | G | | 3' |
| GGT | CTT | TAC | CTT | AA | 5' |

5. Verfahren zur Transportexpression von eukaryotischen, prokaryotischen oder viralen Proteinen in prokaryotischen Zellen, dadurch gekennzeichnet, daß an das Gen für das zu transportierende Proteine an eine DNA-Sequenz nach Anspruch 1 bis 4 koppelt, dieses Fusionsgen in einen Vektor einbaut und damit eine Wirtszelle transformiert, die das exprimierte Protein aus dem Cytoplasma transportiert.

6. Verfahren nach Anspruch 5, dadurch gekennzeichnet, daß die synthetische DNA-Sequenz ein wirtseigenes Signalprotein codiert.

7. Hybridvektor, gekennzeichnet durch eine DNA-Sequenz nach Anspruch 1 bis 4.

**8.** Hybridplasmid, das in einer Eco RI-Schnittstelle die DNA-Sequenz I gemäß Anspruch 4 inseriert enthält.

**9.** Wirtsorganismus, enthaltend einen Vektor nach Anspruch 7 oder 8.

**10.** E. coli, enthaltend einen Vektor nach Anspruch 7 oder 8.

**Patentansprüche für folgenden Vertragsstaat : AT**

**1.** Verfahren zur Herstellung einer synthetischen DNA zum Transport von Proteinen in Expressionssystemen, dadurch gekennzeichnet, daß man eine DNA, die für eine natürliche Signalsequenz codiert, aber eine oder mehrere Schnittstellen für Endonucleasen aufweist, die in der natürlichen DNA nicht enthalten sind, und daß diese DNA am 3'-Ende mindestens 5 bis zu etwa 40 der aminoterminalen Codons desjenigen Strukturgens enthält, das natürlicherweise auf die Signalsequenz folgt, woran sich die Aminosäurefolge des gewünschten Proteins ausschließt.

**2.** Verfahren nach Anspruch 1, dadurch gekennzeichnet, daß die internen Schnittstellen vor und/oder nach der hydrophoben Region eingebaut werden.

**3.** Verfahren nach Anspruch 1 oder 2, dadurch gekennzeichnet, daß eine DNA eingesetzt wird, die für die natürliche Signalsequenz der Alkalischen Phosphatase von E. coli codiert.

**4.** Verfahren zur Transportexpression von eukaryotischen, prokaryotischen oder viralen Proteinen in prokaryotischen Zellen, dadurch gekennzeichnet, daß man das Gen für das zu transportierende Protein an eine DNA-Sequenz nach Anspruch 1 bis 3 koppelt, dieses Fusionsgen in einen Vektor einbaut und damit eine Wirtszelle transformiert, die das exprimierte Protein aus dem Cytoplasma transportiert.

**5.** Verfahren nach Anspruch 4, dadurch gekennzeichnet, daß die synthetische DNA-Sequenz ein wirtseigenes Signalprotein codiert.

**Claims**
**Claims for the following Contracting States : BE, CH, DE, FR, GB, IT, LI, LU, NL, SE**

**1.** A synthetic DNA for the transport of proteins in expression systems, which DNA codes for a natural signal sequence but has one or more cleavage sites for endonucleases which the natural DNA does not contain, and which DNA contains at the 3' end at least 5 and up to about 40 of the amino-terminal coding codons of that structural gene which naturally follows the signal sequence, the amino acid sequence of the required protein being connected thereto.

**2.** A DNA as claimed in claim 1, which contains internal cleavage sites are incorporated upstream and/or downstream of the hydrophobic region.

**3.** A DNA as claimed in claim 1 or 2, which essentially codes for the natural signal sequence of the alkaline phosphatase of E. coli.

**4.**

```
DNA sequence I

Triplet No.                              1     2     3
Amino acid No.                          Met   Lys   Gln
Nucleotide No.                    5           10
Coding strand       5'    AA    TTC    ATG   AAA   CAA
Noncoding strand    3'            G     TAC   TTT   GTT
```

```
     4     5     6     7     8     9     10    11    12    13
     Ser   Thr   Ile   Ala   Leu   Ala   Leu   Leu   Pro   Leu


     15    20          25          30    35          40
     AGC   ACG   ATC   GCA   CTG   GCA   CTC   TTA   CCG   TTA
     TCG   TGC   TAG   CGT   GAC   CGT   GAG   AAT   GGC   AAT


     14    15    16    17    18    19    20    21    22    23
     Leu   Phe   Thr   Pro   Val   Thr   Lys   Ala   Arg   Thr


     45    50          55          60    65          70
     CTG   TTT   ACC   CCG   GTG   ACA   AAA   GCT   CGG   ACC
     GAC   AAA   TGG   GGC   CAC   TGT   TTT   CGA   GCC   TGG


     24    25    26
     Pro   Glu   Met


     75    80          84
     CCA   GAA   ATG   G                 3'
     GGT   CTT   TAC   CTT   AA          5'
```

**5.** A process for the transport expression eukaryotic, prokaryotic or viral proteins in prokaryotic cells, which comprises coupling the gene for the protein which is to be transported onto a DNA sequence as claimed in claim 1 to 4, incorporating this fusion gene into a vector, and transforming therewith a host cell which transports the expressed protein out of the cytoplasm.

**6.** The process as claimed in claim 5, wherein the synthetic DNA sequence codes a signal protein intrinsic to the host.

**7.** A hybrid vector comprising a DNA sequence as claimed in claim 1 to 4.

**8.** A hybrid plasmid containing the DNA sequence I as claimed in claim 4 inserted in an Eco RI cleavage site.

**9.** A host organism containing a vector as claimed in claim 7 or 8.

**10.** E. coli containing a vector as claimed in claim 7 or 8.

**Claims for the following Contracting State : AT**

**1.** A process for the preparation of a synthetic DNA for the transport of proteins in expression systems, which comprises a DNA which codes for a natural signal sequence but has one or more cleavage sites for endonucleases which the natural DNA does not contain, and which DNA contains at the 3' end at least 5 and up to about 40 of the amino-terminal coding codons of that structural gene which naturally follows the signal sequence, the amino acid sequence of the required protein being connected thereto.

2. The process as claimed in claim 1, wherein the internal cleavage sites are incorporated upstream and/or downstream of the hydrophobic region.

3. The process as claimed in claim 1 or 2, wherein a DNA which codes for the natural signal sequence of the alkaline phosphatase of E. coli is used.

4. A process for the transport expression of eukaryotic, prokaryotic or viral proteins in prokaryotic cells, which comprises coupling the gene for the protein which is to be transported onto a DNA sequence as claimed in claim 1 to 3, incorporating this fusion gene into a vector, and transforming therewith a host cell which transports the expressed protein out of the cytoplasm.

5. The process as claimed in claim 4, wherein the synthetic DNA sequence codes a signal protein intrinsic to the host.

**Revendications**
**Revendications pour les Etats contractants suivants : BE, CH, DE, FR, GB, IT, LI, LU, NL, SE**

1. ADN synthétique pour le transport de protéines dans des systèmes d'expression, caractérisé en ce que l'ADN est codant pour une séquence signal naturelle, mais présente un ou plusieurs sites de clivage pour des endonucléases, qui ne sont pas présents dans l'ADN naturel, et que cet ADN renferme, à l'extrémité terminale 3', au moins 5 et jusqu'à environ 40 des codons codants amino-terminaux du gène structural, qui suit naturellement la séquence signal, à laquelle est rattachée la séquence des acides aminés de la protéine souhaitée.

2. ADN selon la revendication 1, caractérisé en ce qu'il contient des sites de clivage internes avant et/ou après la région hydrophobe.

3. ADN selon la revendication 1 ou 2, caractérisé en ce qu'il est surtout condant pour la séquence signal naturelle de la phosphatase alcaline d'E. coli.

**4.**

Séquence I d'ADN

| Triplet N° | | | | | | | | 1 | 2 | 3 |
|---|---|---|---|---|---|---|---|---|---|---|
| Acide aminé N° | | | | | | | | Met | Lys | Gln |
| Nucléotide N° | | | | | | 5 | | · 10 | | |
| Brin codant | | | 5' | AA | TTC | ATG | AAA | CAA | | |
| Brin non codant | | | 3' | | G | TAC | TTT | GTT | | |

| 4 | 5 | 6 | 7 | 8 | 9 | 10 | 11 | 12 | 13 |
|---|---|---|---|---|---|---|---|---|---|
| Ser | Thr | Ile | Ala | Leu | Ala | Leu | Leu | Pro | Leu |

| 15 | 20 | | 25 | | 30 | 35 | | 40 | |
|---|---|---|---|---|---|---|---|---|---|
| AGC | ACG | ATC | GCA | CTG | GCA | CTC | TTA | CCG | TTA |
| TCG | TGC | TAG | CGT | GAC | CGT | GAG | AAT | GGC | AAT |

| 14 | 15 | 16 | 17 | 18 | 19 | 20 | 21 | 22 | 23 |
|---|---|---|---|---|---|---|---|---|---|
| Leu | Phe | Thr | Pro | Val | Thr | Lys | Ala | Arg | Thr |

| 45 | 50 | | 55 | | 60 | 65 | | 70 | |
|---|---|---|---|---|---|---|---|---|---|
| CTG | TTT | ACC | CCG | GTG | ACA | AAA | GCT | CGG | ACC |
| GAC | AAA | TGG | GGC | CAC | TGT | TTT | CGA | GCC | TGG |

| 24 | 25 | 26 |
|---|---|---|
| Pro | Glu | Met |

| 75 | 80 | | 84 | | |
|---|---|---|---|---|---|
| CCA | GAA | ATG | G | | 3' |
| GGT | CTT | TAC | CTT | AA | 5' |

**5.** Procédé d'expression par transport de protéines eucaryotes, procaryotes ou virales dans des cellules procaryotes, caractérisé en ce qu'on couple le gène pour la protéine à transporter à une séquence d'ADN selon l'une quelconque des revendications 1 à 4, et qu'on incorpore ce gène de fusion à un vecteur, avec lequel on transforme une cellule hôte, qui transporte hors du cytoplasme la protéine exprimée.

**6.** Procédé selon la revendication 5, caractérisé en ce que la séquence d'ADN synthétique est codante pour une protéine signal spécifique à l'hôte.

**7.** Vecteur hydride, caractérisé par une séquence d'ADN selon l'une quelconque des revendications 1 à 4.

**8.** Plasmide hydride, qui contient, dans un site de clivage Eco RI, la séquence I d'ADN insérée selon la revendication 4.

**9.** Organisme hôte, contenant un vecteur selon la revendication 7 ou 8.

**10.** E. coli, contenant un vecteur selon la revendication 7 ou 8.

**Revendications pour l'Etat contractant suivant : AT**

**1.** Procédé de fabrication d'un ADN synthétique pour le transport de protéines dans des systèmes d'expression, caractérisé en ce que l'ADN est codant pour une séquence signal naturelle, mais présente un ou plusieurs sites de clivage pour des endonucléases, qui ne sont pas présents dans l'ADN naturel, et que cet ADN renferme, à l'extrémité terminale 3', au moins 5 et jusqu'à environ 40 des codons codants amino-terminaux du gène structural, qui suit naturellement la séquence signal, à laquelle est rattachée la séquence des acides aminés de la protéine souhaitée.

**2.** Procédé selon la revendication 1, caractérisé en ce que les sites de clivage internes sont insérés avant et/ou après la région hydrophobe.

**3.** Procédé selon la revendication 1 ou 2, caractérisé en ce qu'on utilise un ADN qui est codant pour la séquence signal naturelle de la phosphatase alcaline d'E. coli.

**4.** Procédé d'expression par transport de protéines eucaryotes, procaryotes ou virales dans des cellules procaryotes, caractérisé en ce qu'on couple le gène pour la protéine à transporter à une séquence d'ADN selon l'une quelconque des revendications 1 à 3, et qu'on incorpore ce gène de fusion à un vecteur, avec lequel on transforme une cellule hôte, qui transporte hors du cytoplasae la protéine exprimée.

**5.** Procédé selon la revendication 4, caractérisé en ce que la séquence d'ADN synthétique est codante pour une protéine signal spécifique à l'hôte.

17

FIG.1

FIG.5

18

FIG.2

## FIG.3

## FIG.4